(19) Europäisches Patentamt
European Patent Office
Office européen des brevets

(11) Publication number: **0 126 470 B1**

# (12) EUROPEAN PATENT SPECIFICATION

(45) Date of publication of patent specification: 12.04.89

(51) Int. Cl.[4]: C 07 C 93/06

(21) Application number: 84105719.3

(22) Date of filing: 18.05.84

## (54) Process for the preparation of tamoxifen.

(30) Priority: 19.05.83 US 496341
22.02.84 US 580481

(43) Date of publication of application:
28.11.84 Bulletin 84/48

(45) Publication of the grant of the patent:
12.04.89 Bulletin 89/15

(84) Designated Contracting States:
AT BE CH DE FR GB IT LI LU NL SE

(56) References cited:

Chemical Abstracts vol. 97, no. 17, 25 October 1982, Columbus, Ohio, USA; J. BURNS et al. "The preparation of carbon-14- and tritium-labeled 1-4-(2-dimethylaminoethoxy)phenylr-1,2-diphenyl-1-butene ICI 46,474, tamoxifen (Nolvadex)r and the separation of cis-trans isomers.

(73) Proprietor: Bristol-Myers Company
345 Park Avenue
New York New York 10154 (US)

(72) Inventor: Melton, Jack
715 Onondaga Avenue
Syracuse New York (US)

(74) Representative: Kinzebach, Werner, Dr.
Patentanwälte Reitstötter, Kinzebach und Partner Sternwartstrasse 4 Postfach 86 06 49
D-8000 München 86 (DE)

(56) References cited:

JOURNAL OF ORGANIC CHEMISTRY, vol. 46, no. 21, 9 October 1981; L. CASTEDO et al. "Selective reductive carbonyl couplings with titanium", pages 4292-4294

The file contains technical information submitted after the application was filed and not included in this specification

EP 0 126 470 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

This invention relates to a novel and economical process for the preparation of tamoxifen.

Tamoxifen is a known non-steroidal anti-estrogen useful in the treatment of hormone-dependent tumors such as breast cancer. Tamoxifen is the Z or trans isomer of 1,2-diphenyl-1-[4-(2-dimethylaminoethoxy)phenyl]-1-butene and has the formula:

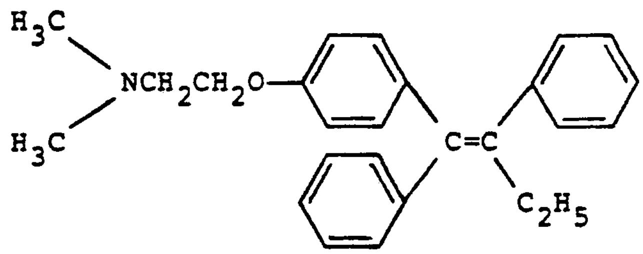

When produced by known processes, the tamoxifen is formed in admixture with the corresponding E or cis isomer. Since the desired biological activity resides only in the Z isomer, the mixture of geometric isomers resulting from chemical synthesis must be separated.

A number of processes are known in the art for the production of tamoxifen and the corresponding E isomer. Thus, U.K. Patent No. 1,013,907 discloses a process which involves dehydration of an alkanol intermediate using an acid catalyst such as HCl to produce a mixture of tamoxifen and the corresponding E isomer. This process is illustrated by the following equation:

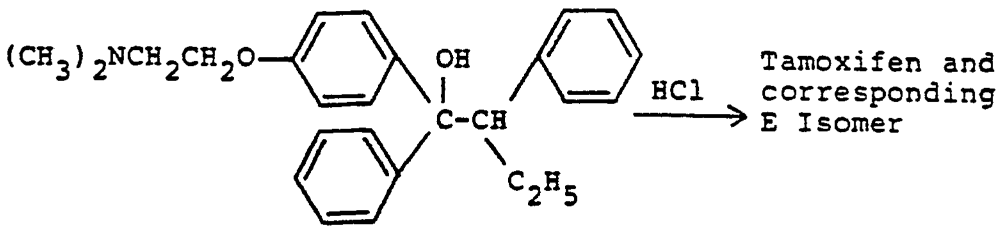


U.K. Patent No. 1,013,907 also discloses a process for the production of tamoxifen and the corresponding E isomer which involves alkylation of a phenolic intermediate. This process is illustrated by the following equation:

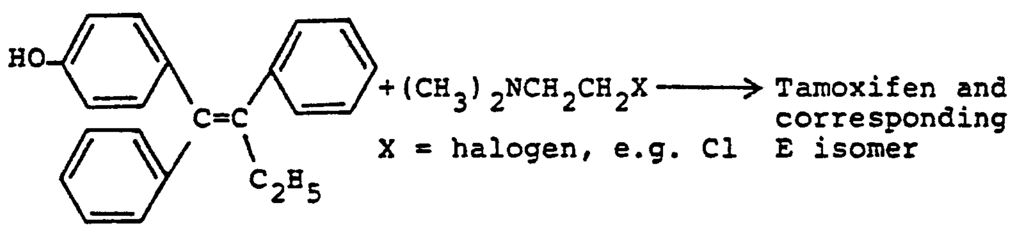


U.K. Patent No. 1,354,939 discloses a process wherein a cyanide intermediate is dehydrogenated by an alkali metal amide to produce a mixture of tamoxifen and the corresponding E isomer. This process is illustrated by the following equation:

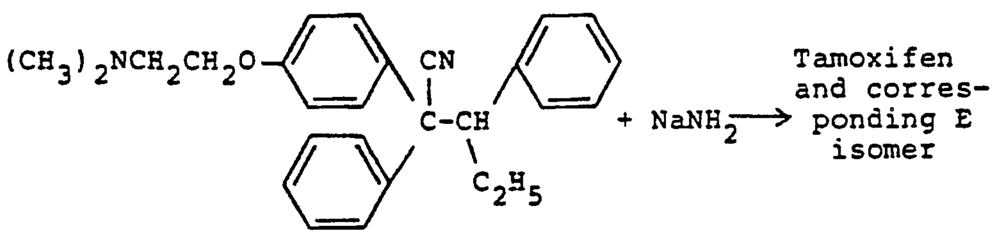


An abstract of Japanese application J5 2122—351, Derwent No. 83880Y/47, discloses the following reaction:

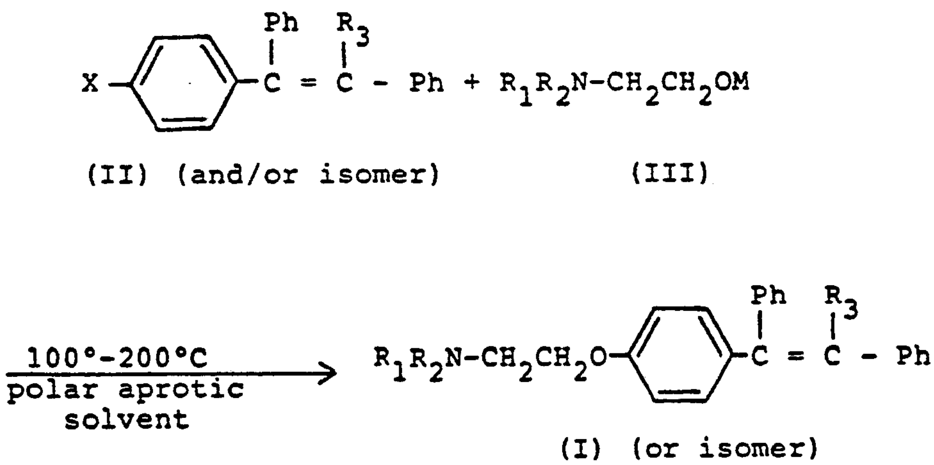


(II) (and/or isomer) (III)

(I) (or isomer)

wherein $R_1$ and $R_2$ are the same or different 1—4 carbon alkyl groups, or $NR_1R_2$ is a heterocyclic group: $R_3$ is a 1—4 carbon alkyl group, X is halogen; and M is H or alkali metal, preferably Na or K.

The use of low valent titanium as a reducing agent to reductively dimerize aldehydes and ketones to olefins is known in the art. Thus, J. E. McMurry, M. P. Fleming, K. L. Kees and L. R. Krepski in *J. Org, Chem.,* Vol. 43, pages 3255-66 (1978) report the use of active titanium metal, produced in a finely divided form by reduction of $TiCl_3$ with either potassium or lithium, to reductively couple carbonyls or form olefins. One reaction disclosed in this paper is illustrated by the following equation:

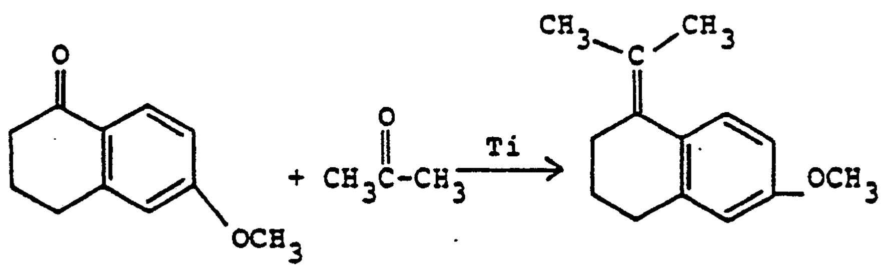


J. E. McMurry and M. P. Fleming in *J. Am. Chem. Soc.,* Vol, 96, pages 4708-9 (1974) disclose the reductive dimerization of a compound having the formula:

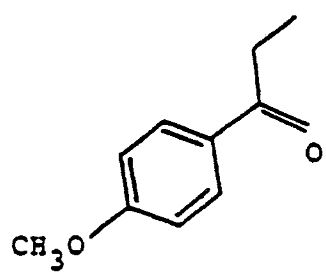

to obtain an olefin having the formula:

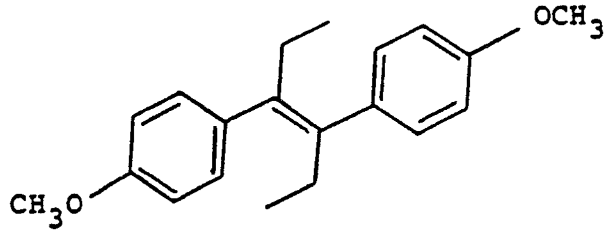

using $LIAlH_4$-$TiCl_3$ as the reducing agent.

L. Castedo, J.M. Saa, R. Suau and G. Tojo in *J. Org. Chem.,* Vol. 46, pages 4292-94 (1981) report that the low-valent titanium reagents used to reductively couple ketones and aldehydes to form olefins also react with other functional groups and that their use had in the past been assumed to be limited to cases where functional groups other than alcohols, ethers and olefins are not present. They report that low-valent titanium species seem in most cases to be active Ti°. These authors found that ester groups could also be

present without being adversely affected by the reaction conditions. Due to the high reactivity of low-valent titanium reagents with nitrogen containing compounds, it would be expected that aldehydes and ketones which are to be reductively coupled to form an olefin by the use of a low valent titanium reagent should be free of amino groups.

Summary Of The Invention

It has now been discovered that a mixture of tamoxifen and the corresponding E isomer can be produced by a process which comprises the reductive coupling of 4-(beta-dimethylaminoethoxy)benzophenone, or an acid salt thereof, and propiophenone, said coupling being induced in the presence of a low valent titanium reagent. The reaction which occurs is illustrated by the following equation:

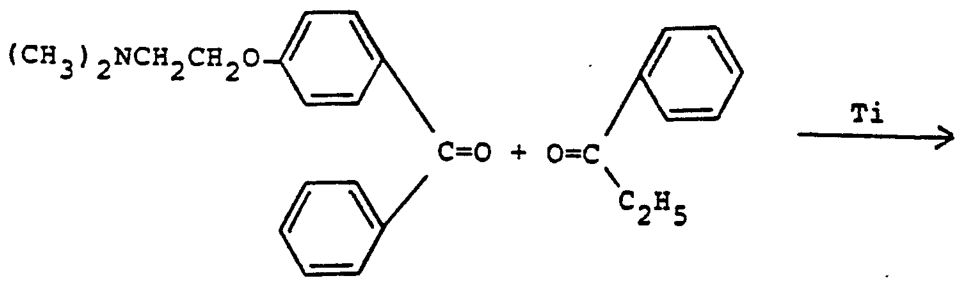


Tamoxifen + corresponding cis isomer + $TiO_2$

Tamoxifen can be separated from the resultant mixture of cis and trans isomers by conventional means, e.g., be selective crystallization.

Detailed Description Of The Invention

The low valent titanium reagent can be prepared by procedures well known in the art, i.e., by reduction of $TiCl_3$ or $TiCl_4$ with Mg, Zn, K, Li or $LiAlH_4$. The $TiCl_3$ or $TiCl_4$ is added to a common ether solvent such as tetrahydrofuran, diethyl ether, dioxane or dimethylethane before, after or concurrently with the Mg, Zn, K, Li or $LialH_4$. In the case of $TiCl_4$, it is preferred to first dissolve it in a hydrocarbon solvent such as toluene and the to add the ether solvent to this solution.

The mixture of $TiCl_3$ or $TiCl_4$ and metal in the solvent is heated to reflux, preferably for from 4—20 hours to form a slurry of low valent activated titanium reagent. The reagent may be used in the subsequent reductive coupling reaction as formed, i.e., it need not be isolated from the slurry. It may be used while still hot or it may be cooled and stored for a period of hours before using. However, it is preferred to use the reagent within 24 hours of its preparation, since it is likely to start to deteriorate after that time, thus resulting in lowered yields of tamoxifen and the corresponding cis isomer.

The reductive coupling reaction is preferably conducted in an inert solvent, e.g., an ether solvent such as tetrahydrofuran, diethl ether, dioxane or dimethoxyethane. The 4-(beta-dimethylaminoethoxy)benzophenone may be used in the form of the free base or an acid salt. Examples of suitable acids for salt formation are hydrochloric, sulfuric, phosphoric, acetic, citric, oxalic, malonic, salicyclic, malic, fumaric, succinic, methanesulfonic, and the like.

The 4-(beta-dimethylaminoethoxy)benzophenone can be added to the low valent activated titanium reagent prior to the addition of the propiophenone in which case it will form a complex with the low valent activated titanium reagent. This complex can then be titrated by the slow addition of propiophenone, i.e., the low valent activated titanium reagent 4-(beta-dimethylaminoethoxy)benzophenone complex will react with the propiophenone as soon as the propiophenone is added. Alternatively, the 4-(beta-dimethylaminoethoxy)benzophenone can be added to the activated titanium reagent in admixture with the propiophenone in which case it is preferred to use from 1 to 1.5 mole of propiophenone per mole of 4-(beta-dimethylaminoethoxy)benzophenone. The propiophenone should not be added first to the low valent activated titanium reagent since it would immediately couple with itself. At least one mole of low valent activated titanium reagent is preferably present for each mole of propiophenone and 4-(beta-dimethylaminoethoxy)benzophenone. The reductive coupling reaction is preferably conducted by heating the reaction.

After the reductive coupling reaction is complete, the mixtrue of tamoxifen and its E isomer can be recovered from the reaction mixture by conventional techniques. The tamoxifen can then be separated from the E isomer by known procedures, i.e., by chromatography or fractional crystallization.

The herein described reductive coupling procedure for the production of tamoxifen is more efficient and economical than any of the prior art processes for the production of tamoxifen.

The following example illustrates the process of this invention.

Example

I Preparation of Ketone Mixture

4-(Beta-dimethylaminoethoxy)benzophenone Hcl (50.0 g, 0.164 mole) was suspended in 600 ml of methylene chloride and 600 ml of water at room temperature (initial pH of 2.9). The pH was raised to 12.2 by the dropwise addition of 14 ml of 50% (w/w) NaOH. The two phase system was stirred for 20 minutes at 20°C, with no change in pH. The phases were separated, and the aqueous was discarded. The organic phase was vacuum concentrated at 25°C to an oil. The oil was dissolved in 200 ml of THF (tetrahydrofuran) and reconcentrated under vacuum at 30°C. The oily residue was then dissolved in 150 ml of THF. Propiophenone (30.9 g, 0.245 mole) was added and the solution of ketones was charges into a 250 ml addition funnel.

II Preparation of Low Valent Activated
Titanium Reagent, Ti

Titanium trichloride (164.6 g, 1.06 mole) was added to 1,000 ml of THF, and the slurry was mechanically stirred under a blanket of nitrogen for 15 minutes at 30°C. Powdered zinc (180 g, 2.75 mole) was added in one portion, and the slurry was heated to reflux (67°C) for 4 hours, then allowed to cool to 60°C.

III Ketone Coupling Reaction

The solution of ketones from step I was added in 15 minutes to the warm, well stirred Ti slurry, rinsed forward with a 100 ml portion of THF, and brought back to reflux for 1 hour and 30 minutes.

IV Isolation of Crude HC1 Salt
of Tamoxifen and E Isomer

The reaction was cooled to 25°C and poured into a 5 liter, round bottom flask charged with 2 liters of distilled water, and digested at 40-50°C for 30 minutes under a blanket of nitrogen. The slurry was filtered through Dicalite® and this filter cake was washed 2 times with 100 ml portions of tetrahydrofuran. The tetrahydrofuran was removed by distillation at reduced pressure (about 15 mm Hg). The concentrate was extracted two times with 1250 ml portions of methylene chloride. The combined methylene chloride extracts were washed with two 750 ml portions of water. The methylene chloride solution was then stirred for 15 minutes with water (1000 ml) and the pH was adjusted to 12.7. The phases were separated with the aqueous discarded. The methylene chloride phase was concentrated to an oil. At this point, the ratio of Z to E isomer was about 2:1. The oil was dissolved in 200 ml of methyl isobutyl ketone, and concentrated to an oil. This residue (free of methylene chloride) was dissolved in 1 liter of methyl isobutyl ketone, washed with 1 liter of distilled water and the pH was readjusted to 12 with 50% sodium hydroxide solution. The phases were separated and the organic phase retained. The methyl isobutyl ketone solution was vacuum concentrated to a volume of 750 ml. A 3 liter round bottom flask equipped with mechanical stirring was charged with the methyl isobutyl ketone solution of tamoxifen free base and the corresponding E isomer. To this solution, 13.7 ml of 12 *N* hydrochloric acid was added in 5 minutes to form tamoxifen hydrochloride and the corresponding E isomer. The slurry that formed was chilled to 0—5°C and held for 18 hours, filtered, washed with 350 ml of methyl isobutyl ketone (0°C), and dried in a vacuum oven at 50—80°C for 18 hours to yield 34.2 g., .084 mole, 51.2% yield of the hydrochloride salt of tamoxifen and the corresponding E isomer of >85% purity. The Z:E isomer ratio was 97.5:1.

**Claims**

1. A process for the production of a mixture of tamoxifen and the corresponding E isomer which comprises the reduction coupling of 4-(beta-di-methylaminoethoxy)benzophenone or an acid salt thereof and propiophenone, said coupling being induced in the presence of a low valent titanium compound.

2. A process as defined in claim 1 wherein said low valent titanium reagent is prepared by reduction of $TiCl_3$ or $TiCl_4$ with magnesium, zinc, potassium, lithium or lithium aluminum hydride in an inert solvent.

3. A process as defined in claim 2 wherein said $TiCl_3$ or $TiCl_4$ and reducing agent in said solvent are heated to reflux for from four to 24 hours to form a slurry of low valent activated titanium reagent.

4. A process as defined in claim 3 wherein said low valent activated titanium reagent is used in said reductive coupling reaction without isolating it from the inert solvent in which it is formed.

5. A process as defined in claim 3 wherein said low valent activated titanium reagent is used in said reductive coupling reaction within 24 hours of its preparation.

6. A process as defined in claim 3 wherein said low valent activated titanium reagent is prepared by contacting $TiCl_3$ with powdered zinc metal in tetrahydrofuran.

7. A process as defined in claim 1 wherein said reductive coupling reaction is conducted in an inert solvent.

8. A process as defined in claim 7 wherein said inert solvent is an ether.

9. A process as defined in claim 8 wherein said ether is selected from the group consisting of tetrahydrofuran, diethylether, dioxane and dimethoxyethane.

10. A process as defined in claim 7 wherein said 4-(beta-dimethylaminoethoxy)benzophenone is added to said low valent titanium reagent, said low valent titanium reagent being in an inert solvent, prior to the

addition of said propiophenone whereby a complex is formed and said complex is then titrated by the slow addition of propiophenone.

11. A process as defined in claim 7 wherein a mixture of 4-(beta-dimethylaminoethoxy)benzophenone and propiophenone is added to said low valent titanium reagent, said low valent titanium reagent being in an inert solvent.

12. A process as defined in claim 11 wherein the mixture comprises from 1 to 1.5 mole of propiophenone per mole of 4-(beta-dimethylaminoethoxy)benzophenone.

13. A process as defined in claim 7 wherein at least one mole of said low valent titanium reagent is present for each mole of propiophenone and 4-(beta-dimethylaminoethoxy)benzophenone.

14. A process as defined in claim 7 wherein said reductive coupling reaction is conducted by heating the reaction mixture to reflux.

15. A process as defined in any one of claims 1, 7, 8, 10, 11, 13 or 14, wherein the tamoxifen is subsequently separated from the corresponding E isomer.

## Patentansprüche

1. Verfahren zur Herstellung einer Mischung von Tamoxifen und dem entsprechenden E-Isomer, welches die reduktive Umsetzung von 4-(β-Dimethylaminoethoxy)benzophenon oder einem Säuresalz davon mit Propiophenon umfaßt, wobei die Umsetzung in Anwesenheit einer niederwertigen Titanverbindung induziert wird.

2. Verfahren nach Anspruch 1, worin das niederwertige Titanreagenz durch Reduktion von $TiCl_3$ oder $TiCl_4$ mit Magnesium-, Zink-, Kalium-, Lithium- oder Lithiumaluminiumhydrid in einem inerten Lösungsmittel hergestellt wird.

3. Verfahren nach Anspruch 2, worin $TiCl_3$ oder $TiCl_4$ und das Reduktionsmittel im Lösungsmittel unter Rückfluß für 4 bis 24 Stunden erhitzt werden, wobei sich eine Aufschlämmung des niederwertigen aktivierten Titanreagenzes bildet.

4. Verfahren nach Anspruch 3, worin das niederwertige aktivierte Titanreagenz in der reduktiven Umsetzung verwendet wird, ohne daß es aus dem inerten Lösungsmittel, in welchem es gebildet wird, isoliert wird.

5. Verfahren nach Anspruch 3, worin das niederwertige aktivierte Titanreagenz in der reduktiven Umsetzung innerhalb von 24 Stunden nach seiner Herstellung verwendet wird.

6. Verfahren nach Anspruch 3, worin das niederwertige aktivierte Titanreagenz hergestellt wird, indem $TiCl_3$ mit Zinkmetallpulver in Tetrahydrofuran in Kontakt gebracht wird.

7. Verfahren nach Anspruch 1, worin die reduktive Umsetzung in einem inerten Lösungsmittel durchgeführt wird.

8. Verfahren nach Anspruch 7, worin das inerte Lösungsmittel Ether ist.

9. Verfahren nach Anspruch 8, worin der Ether ausgewählt wird unter Tetrahydrofuran, Diethylether, Dioxan und Dimethoxyethan.

10. Verfahren nach Anspruch 7, worin 4-(β-Dimethylaminoethoxy)benzophenon zum niederwertigen Titanreagenz, welches sich in einem inerten Lösungsmittel befindet, vor der Zugabe von Propiophenon gegeben wird, wodurch sich ein Komplex bildet, der dann durch langsame Zugabe von Propiophenon titriert wird.

11. Verfahren nach Anspruch 7, worin eine Mischung von 4-(β-Dimethylaminoethoxy)benzophenon und Propiophenon zu dem niederwertigen Titanreagenz gegeben wird, wobei das niederwertige Titanreagenz sich in einem inerten Lösungsmittel befindet.

12. Verfahren nach Anspruch 11, worin die Mischung 1 bis 1,5 Mol Propiophenon pro Mol 4-(β-Dimethylaminoethoxy)benzophenon umfaßt.

13. Verfahren nach Anspruch 7, worin mindestens ein Mol des niederwertigen Titanreagenzes pro Mol Propiophenon und 4-(β-Dimethylaminoethoxy)benzophenon vorhanden ist.

14. Verfahren nach Anspruch 7, worin die reduktive Umsetzung durch Erhitzen des Reaktionsgemisches bis zum Rückfluß ausgeführt wird.

15. Verfahren nach einem der Ansprüche 1, 7, 8, 10, 11, 13 oder 14, worin Tamoxifen anschließend von dem entsprechenden E-Isomer getrennt wird.

## Revendications

1. Un procédé de production d'un mélange de tamoxifène (1-p-béta-diméthylaminoéthoxyphényl-trans-1,2-diphénylbut-1-ème) et de l'isomère E correspondant qui comprend le couplage réducteur de la 4-(béta-diméthylaminoéthoxy)benzophénone ou d'un sel acide en dérivant et de propiophénone, ce couplage étant initié en présence d'un composé de valence faible du titane.

2. Un procédé tel que défini dans la revendication 1, dans lequel le réactif de valence faible du titane précité est préparé par réduction de $TiCl_3$ ou $TiCl_4$ par l'hydrure de lithium-aluminium, magnésium, zinc, potassium ou lithium dans un solvant inerte.

3. Un procédé tel que défini dans la revendication 2, dans lequel lesdits $TiCl_3$ ou $TiCl_4$ et agent

réducteur dans ledit solvant sont chauffés au reflux pendant 4 à 24 heures pour former une suspension de réactif de titane activé de valence faible.

4. Un procédé tel que défini dans la revendication 1, dans lequel le réactif de titane activé de faible valence est utilisé dans ladite réaction de couplage réducteur sans nécessiter de l'isoler du solvant inerte dans lequel il se forme.

5. Un procédé tel que défini dans la revendication 3, dans lequel le réactif de titane activé de faible valence précité est utilisé dans ladite réaction de couplage réducteur dans les 24 heures de sa préparation.

6. Un procédé tel que défini dans la revendication 3, dans lequel le réactif de titane activé de faible valence est préparé par contact de $TiCl_3$ avec du zinc métallique pulvérulent dans le tétrahydrofurane.

7. Un procédé tel que défini dans la revendication 1, dans lequel ladite réaction de couplage réducteur est conduite dans un solvant inerte.

8. Un procédé tel que défini dans la revendication 7, dans lequel le solvant inerte précité est un éther.

9. Un procédé tel que défini dans la revendication 8, dans lequel l'éther précité est choisi dans le groupe comprenant tetrahydrofurane, diéthyléther, dioxane et diméthoxyéthane.

10. Un procédé tel que défini dans la revendication 7, dans lequel la 4-(béta-diméthylaminoéthoxy)benzophénone précitée, est ajoutée au réactif à base de titane de valence faible précité, le réactif à base de titane étant dans un solvant inerte, préalablement à l'addition de la propiophénone précitée, un complexe se formant, et étant alors titré par lente addition de propiophénone.

11. Un procédé tel que défini dans la revendication 7, dans lequel un mélange de 4-(béta-diméthylaminoéthoxy)benzophénone et de propiophénone est ajouté au réactif de titane de faible valence, ledit réactif à base de titane de valence faible étant dans un solvant inerte.

12. Un procédé tel que défini dans la revendication 11, dans lequel le mélange comprend de 1 à 1,5 mole de propiophénone par mole de 4-(béta-diméthylaminoéthoxy)benzophénone.

13. Un procédé tel que défini dans la revendication 7, dans lequel au moins une mole dudit réactif à base de titane de valence faible est présent par mole de propiophénone et de 4-(béta-diméthylaminoéthoxy)benzophénone.

14. Un procédé tel que défini dans la revendication 7, dans lequel ladite réaction de couplage réducteur est effectuée par chauffage du mélange réactif à la temperature du reflux.

15. Un procédé tel que défini dans une quelconque des revendications 1, 7, 8, 10, 11, 13 ou 14, dans lequel le tamoxifène est ultérieurement séparé de l'isomère E correspondant.